(19) **Europäisches Patentamt / European Patent Office / Office européen des brevets**

(11) **EP 2 714 848 B1**

(12) **FASCICULE DE BREVET EUROPEEN**

(45) Date de publication et mention
de la délivrance du brevet:
**22.05.2019 Bulletin 2019/21**

(21) Numéro de dépôt: **12729686.1**

(22) Date de dépôt: **31.05.2012**

(51) Int Cl.:
*C10G 2/00* *(2006.01)*  *B01J 21/06* *(2006.01)*
*B01J 27/22* *(2006.01)*  *B01J 27/224* *(2006.01)*
*B01J 37/02* *(2006.01)*  *B01J 23/745* *(2006.01)*
*B01J 23/75* *(2006.01)*  *B01J 23/78* *(2006.01)*
*B01J 23/83* *(2006.01)*  *B01J 23/89* *(2006.01)*
*B01J 35/00* *(2006.01)*  *B01J 35/10* *(2006.01)*
*B01J 37/00* *(2006.01)*  *C07C 1/04* *(2006.01)*

(86) Numéro de dépôt international:
**PCT/FR2012/051224**

(87) Numéro de publication internationale:
**WO 2012/164231 (06.12.2012 Gazette 2012/49)**

(54) **PROCÉDÉ CATALYTIQUE POUR LA CONVERSION D'UN GAZ DE SYNTHÈSE EN HYDROCARBURES**

KATALYTISCHES VERFAHREN ZUR UMWANDLUNG EINES SYNTHESEGASES IN KOHLENWASSERSTOFFE

CATALYTIC PROCESS FOR THE CONVERSION OF A SYNTHESIS GAS TO HYDROCARBONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.06.2011 FR 1101704**
**02.08.2011 FR 1157096**

(43) Date de publication de la demande:
**09.04.2014 Bulletin 2014/15**

(73) Titulaires:
• **Sicat LLC**
  **New York, New York 10022 (US)**
• **Total SA**
  **92400 Courbevoie (FR)**
• **Centre National de la Recherche Scientifique**
  **75794 Paris Cedex 16 (FR)**
• **University Of Strasbourg**
  **67081 Strasbourg Cedex (FR)**

(72) Inventeurs:
• **LUCK, Francis**
  **93160 Noisy-le-grand (FR)**
• **PHAM, Charlotte**
  **67000 Strasbourg (FR)**
• **NGUYEN, Patrick**
  **67000 Strasbourg (FR)**
• **PHAM-HUU Cuong**
  **67100 Strasbourg (FR)**
• **DE TYMOWSKI Benoît**
  **75013 Paris (FR)**

(74) Mandataire: **Schmidt, Martin Peter**
**IXAS Conseil**
**15, rue Emile Zola**
**69002 Lyon (FR)**

(56) Documents cités:
**EP-A1- 1 985 361       US-A- 5 648 312**
**US-A1- 2005 002 838**

EP 2 714 848 B1

**Description**

**Domaine technique de l'invention**

La présente invention concerne la synthèse Fischer-Tropsch, et plus particulièrement les catalyseurs pour cette réaction.

**Etat de la technique**

La synthèse de Fischer - Tropsch (appelé ici aussi « SFT »), inventée en 1923, transforme un mélange de monoxyde de carbone (CO) et d'hydrogène ($H_2$) appelé « gaz de synthèse », obtenu à partir de matières premières carbonées (notamment le charbon), en un mélange d'hydrocarbures gazeux et liquides. La réaction est exothermique et procède en présence d'un catalyseur. Depuis quelque temps, on observe un regain d'intérêt pour ce procédé. Les raisons sont multiples : le prix élevé du pétrole brut, les contraintes législatives sur les carburants et les émissions qu'engendre leur combustion, des considérations géopolitiques visant à réduire la dépendance énergétique (surtout aux Etats-Unis qui disposent de la plus grande réserve de charbon au monde), les besoins énergétiques croissants de la Chine et de l'Inde (deux pays qui disposent de peu de ressources en pétrole, mais d'énormes ressources de charbon), la possibilité d'utiliser de la biomasse (la seule ressource carbonée inépuisable) pour la SFT.

Ainsi, de très nombreuses recherches ont porté sur la géométrie du réacteur, sur le catalyseur, et sur les conditions de réaction. En particulier, le catalyseur a fait l'objet de très nombreuses recherches, tant pour la composition et structure microscopique de la phase active que pour le support du catalyseur.

D'une manière générale, il existe deux grandes familles de procédés, exploitant deux types de catalyseurs différents, qui ont été utilisés pour la SFT: Les procédés de type slurry (utilisant un catalyseur à base de cobalt ou de fer) et les procédés en lit fixe (dont la plupart utilisent un catalyseur à base de cobalt).

Le catalyseur pour la SFT doit successivement casser des liaisons (dissociation de CO et de $H_2$) puis en reformer d'autres (hydrogénation et croissance de chaîne par couplage C-C à la surface du métal). On sait que les métaux permettant la meilleure dissociation et le meilleur couplage sont ceux du groupe 8 (Co, Fe et Ru) ainsi que le Ni. Le choix du métal est déterminant à la fois pour la sélectivité et pour le prix du catalyseur.

On sait que les catalyseurs à base de fer forment une grande quantité de $CO_2$ via la réaction appelée « *water gas shift* » ($CO+H_2O \Leftrightarrow CO_2+H_2$). De tels catalyseurs ne conviennent pas pour le GTL (« Gas *To* Liquids ») mais plutôt pour des gaz de synthèse issus de la gazéification du charbon ou de la biomasse (« *Coal to Liquids »*, *« Biomass to Liquids »,* faible ratio $H_2$/CO). Par ailleurs ces catalyseurs produisent des oléfines linéaires aussi bien que des alcools, aldéhydes et cétones. Le ruthénium est le plus actif mais aussi le plus cher. C'est donc le cobalt, qui présente le meilleur équilibre entre stabilité, activité et prix qui est majoritairement utilisé.

Les catalyseurs à base de cobalt connus comprennent en général une phase active comprenant entre 10 et 30% massiques de cobalt, un second métal comme promoteur (typiquement Pt, Ru ou Pd) et un dopant structurel (typiquement Zr, Ba, La), déposés sur un support à base d'oxyde réfractaire à haute surface spécifique (souvent modifié par un dopant structurel). On sait que la taille des particules de Co est déterminante pour l'activité et la sélectivité du catalyseur FT. Au-dessous de 8 nm, l'activité et la sélectivité diminuent fortement, la taille optimale des particules de cobalt semble se situer autours de 8 à 10 nm.

Le cobalt doit se trouver sous sa forme métallique, et donc la réduction de son oxyde doit être complète. Or, sur les supports à base d'oxydes (typiquement $SiO_2$, $Al_2O_3$, $TiO_2$ ou une combinaison de ces derniers), les petites particules de Co sont difficilement réductibles du fait de la forte interaction métal/support. Pour cette raison, un promoteur (typiquement Ru, Pt, Pd) est associé à la phase active pour favoriser sa réduction. Ces métaux nobles sont connus pour former des particules bimétalliques et des alliages qui influent sur la sélectivité et l'activité et sur la dispersion du Co ; par ailleurs ils inhibent la désactivation. Cependant, une trop grande quantité de promoteur peut bloquer les sites actifs du Co. L'addition de zirconium à un support de catalyseur à base d'alumine favorise la réduction des oxydes de cobalt déposés sur ce support ; le Zr limite la formation d'aluminates. Les particules de cobalt doivent être convenablement dispersées sur le support. Si la dispersion des particules est mauvaise, les sites actifs du cobalt ne sont pas employés de manière optimale. En revanche une bonne dispersion des particules de cobalt entraine une augmentation de la conversion catalytique du monoxyde de carbone.

D'une manière générale, le support de catalyseur joue un rôle très important dans la SFT. Sur le plan fonctionnel, il doit pouvoir disperser les particules de Co, avoir une très bonne résistance mécanique (surtout en mode *« slurry »*), et apporter une stabilité thermique aux particules de Co.

Par ailleurs, le support doit résister aux conditions hydrothermales ainsi qu'aux acides et alcools formés lors de la synthèse Fischer-Tropsch. Or, aussi bien l'alumine que le dioxyde de titane et la silice ne montrent pas une stabilité élevée en présence de pressions partielles élevées d'eau, d'alcools et d'acides formés par la SFT. De tels supports doivent donc être stabilisés pour pallier les problèmes de solubilité.

Le brevet US 6,875,720 enseigne l'amélioration de la résistance de l'alumine par protection avec du silicium, du zirconium et du titane ; un support à base de dioxyde de titane (référence P25 ex-Degussa), après transformation en rutile et mise en forme par atomisation est protégé par l'addition de Si.

La demande de brevet US 2005/0124490 décrit l'utilisation d'une alumine promue à la silice pour accroitre sa résistance aux acides et aux alcools. Le brevet US 7,163,963 décrit le dopage d'une alumine aux terres rares (notamment La ou Nd) pour accroitre la résistance chimique du support.

Les supports à base d'alumine et de silice sont le plus couramment utilisés, bien qu'un support à base de $TiO_2$ présente également d'excellentes performances. Il est habituellement admis par l'homme du métier que le $TiO_2$ mis en forme (notamment sous la forme de granules par extrusion, ou de microsphères par atomisation) présente une résistance mécanique inférieure aux supports oxydes précités. Le brevet US 5,484,757 décrit un procédé de fabrication de supports de catalyseur en $TiO_2$ à tenue mécanique améliorée.

Dans l'article paru dans Journal of Catalysis 236 (2005) 139-152, il a été démontré que des catalyseurs à base de cobalt et cobalt promu au rhénium déposés sur du $TiO_2$ rutile de faible surface spécifique ayant des macropores (diamètre 790 nm) étaient plus sélectifs que ses homologues préparés sur $\gamma$-alumine et silice. Les auteurs ont noté que la distribution poreuse du support a une très grande influence sur la taille et la morphologie des particules de cobalt.

Pour soustraire l'influence de la nature chimique du support la même équipe de recherche a étudié l'influence de la taille des pores de différents supports en alumine envers la sélectivité $C_{5+}$ (c'est-à-dire la sélectivité envers des hydrocarbures possédant au moins 5 atomes de carbone). Il a été clairement démontré que plus le diamètre moyen des pores est grand meilleure sera la sélectivité en $C_{5+}$.

Dans l'article Journal of Catalysis 259 (2008) 161-164, les auteurs se sont affranchis de la taille des particules de cobalt et ont synthétisé plusieurs séries de catalyseurs préparés sur $\gamma$-$Al_2O_3$ et $\alpha$-$Al_2O_3$. Les catalyseurs préparés sur alumine alpha se sont montrés systématiquement plus sélectifs que leurs homologues préparés sur alumine gamma. Dans l'article Applied Catalysis A : General 154 (2010) 162-182, les auteurs ont observé l'influence de la phase cristallographique d'une même alumine mais ayant subi des traitements thermiques à différentes températures. Plus la taille des pores est importante, plus la sélectivité en $C_{5+}$ est améliorée. Les catalyseurs préparés sur une alumine alpha de faible volume poreux et de faible surface spécifique se sont montrés plus sélectifs que leurs homologues préparés sur alumine gamma.

Dans le brevet US 7,351,679 une $\gamma$-alumine a été traitée thermiquement pour avoir au moins 10% en masse d'alumine-$\alpha$ et une surface spécifique inférieure à 50 m$^2$/g. Ce document fait référence à l'article « Selectivity control and catalyst design in the Fischer-Tropsch synthesis : sites, pellets and reactors » paru dans Advances in Catalysis, Vol. 39, 1993, p 221-302, qui décrit qu'un maximum de sélectivité $C_{5+}$ est atteint en concevant un support (ou grain de catalyseur) ayant une diffusion réactifs/produits optimale. En effet, dans les larges pores, les alpha-oléfines auront tendance à désorber avant de prolonger leur chaîne de carbone. Dans les pores étroits, la diffusion de CO sera ralentie, conduisant à un défaut de CO au sein des particules de catalyseur et à un enrichissement en hydrogène. Cet enrichissement va conduire à une hydrogénation des oléfines, diminuant la sélectivité en hydrocarbures plus lourds.

Un paramètre $\chi$, fonction de la taille des particules, de la porosité, de la charge de cobalt et de la dispersion de cobalt, a été introduit pour exprimer la résistance diffusionnelle dans une particule de catalyseur ; il est défini par :

$$\chi = R_o{}^2 \phi \Theta / r_p$$

avec :

$R_o$ : diamètre de la particule de catalyseur (m)
$\phi$ : porosité du catalyseur
$\Theta$ : densité des sites catalystiques (site/m$^2$)
$r_p$ : rayon moyen des pores (m)

L'optimum de la sélectivité $C_{5+}$ est donné pour une valeur de $\chi$ comprise entre 500 et 1000 x 10$^{16}$ m$^{-1}$. Pour faire varier $\chi$, le paramètre le plus sensible est la taille macroscopique du grain de catalyseur. Or en lit fixe, le catalyseur doit avoir une taille supérieure au millimètre pour limiter la perte de charge. En lit fluidisé, la valeur de $\chi$ est inférieure à l'optimum car on ne peut pas faire varier indépendamment la dispersion du cobalt, le volume poreux et la charge de cobalt.

Le document US 7,351,679 précité propose un catalyseur au cobalt sur une alumine de basse surface spécifique (< 50 m$^2$/g) ayant de larges pores. Une telle alumine peut être obtenue en traitant thermiquement une alumine gamma entre 700 et 1300°C pendant 1 à 15 heures. Idéalement, le support doit contenir au moins 80% d'alumine alpha et une surface spécifique inférieure à 30 m$^2$/g. Un tel support permet de synthétiser un catalyseur pour la réaction de Fischer-Tropsch plus sélectif en $C_{5+}$. Cependant, la faible surface spécifique du support ne permet pas de déposer une grande quantité de cobalt et les inventeurs se limitent à 12% en masse de cobalt pour ne pas affecter la sélectivité $C_{5+}$.

L'homme du métier est donc confronté à un équilibre entre surface spécifique, diamètre des pores et volume poreux : plus le matériau contient d'alumine alpha, moins sa surface spécifique sera élevée et plus la taille des pores sera grande ; inversement, plus le matériau contient de phase gamma alumine, plus sa surface spécifique sera importante, en revanche plus les pores seront étroits. Une grande surface spécifique permet une meilleure dispersion du cobalt ce qui se traduit par une meilleure activité (meilleure conversion du monoxyde de carbone) et de larges pores conduisent à une augmentation de sélectivité $C_{5+}$.

Il existe donc un besoin de disposer d'un support pouvant résister aux conditions hydrothermales drastiques de la synthèse Fischer-Tropsch, ayant suffisamment de surface spécifique pour disperser la phase active et de larges pores pour améliorer la sélectivité en $C_{5+}$.

Par ailleurs, il existe également un besoin d'avoir un support à base de $TiO_2$, connu par l'homme de l'art pour améliorer la sélectivité en $C_{5+}$, et qui est suffisamment stable mécaniquement pour être utilisé dans la réaction de Fischer-Tropsch.

Un autre problème des catalyseurs Fischer-Tropsch est lié à leur résistance mécanique. En effet, l'une des exigences d'un catalyseur Fischer-Tropsch utilisé dans un réacteur à lit fluidisé est que les grains de catalyseur doivent maintenir leur intégrité aussi longtemps que possible. En effet, l'une des causes de la diminution de la durée de vie des catalyseurs Fischer-Tropsch est la perte de cobalt actif par attrition. Par ailleurs, les produits de réaction peuvent être pollués par les fines poudres de catalyseurs formées par leur dégradation mécanique. Bien que donnant de très bons résultats notamment en sélectivité $C_{5+}$, les supports de $TiO_2$ ne sont pas envisagés en lit fluidisé par manque de résistance à l'attrition. La silice ne semble pas apporter de satisfaction et seule l'alumine modifiée supporte les contraintes du procédé. Comme mentionné ci-dessus, il n'existe cependant pas de matériau qui présente à la fois une grande surface spécifique, c'est à dire au moins supérieure à 30 $m^2$/g et préférablement supérieure à 50 $m^2$/g ayant en plus de larges pores (supérieurs à 30 nm), et une résistance mécanique acceptable. De plus, compte tenu des résultats prometteurs obtenus avec les catalyseurs de SFT sur support $TiO_2$, concernant plus particulièrement la sélectivité en $C_{5+}$, il serait avantageux d'avoir un support de catalyseur avec une surface chimique identique ou proche de celle du $TiO_2$, avec préférentiellement une surface spécifique supérieure à 30 $m^2$/g et surtout des propriétés mécaniques permettant au catalyseur d'être employé dans un réacteur à lit fixe et dans un réacteur à lit fluidisé. US 5,648,312 divulgue un procédé Fischer-Tropsch utilisant un catalyseur comprenant une phase active de titane déposée sur un support consistant en un mélange de SiC et silice.

## Objets de l'invention

Le but de la présente invention est de fournir un catalyseur pour la réaction de Fischer-Tropsch extrêmement actif avec une forte sélectivité en $C_{5+}$.

Un autre but de la présente invention est de fournir un support de catalyseur, utilisable notamment pour la réaction de Fischer-Tropsch, présentant une grande robustesse mécanique pour permettre son utilisation dans un lit fluidisé, réputé pour générer de dommages importants aux particules de catalyseurs.

Enfin, un autre but de l'invention est de fournir un catalyseur qui offre la possibilité de faciliter la récupération de la phase active et les éléments dopants des catalyseurs usagés.

Par la présente invention, les inventeurs apportent une solution aux problèmes posés en proposant un matériau à base de carbure permettant de modifier de manière quasi indépendante la surface spécifique, le volume macroporeux et la distribution macroporeuse. Ce type de matériau est particulièrement adapté pour réaliser un catalyseur pour la réaction de Fischer-Tropsch, qui pourra être utilisé aussi bien en lit fluidisé qu'en lit fixe. Selon l'invention, ce type de matériau contient au moins une phase de carbure de silicium (SiC), la phase préférée pour le carbure de silicium étant la phase beta, et au moins une phase comportant du titane sous la forme du carbure de titane (TiC) et/ou de l'oxyde de titane ($TiO_2$).

Ce type de matériau peut être soumis à un traitement thermique oxydant qui conduit à l'oxydation partielle ou totale des carbures, et plus particulièrement du TiC en $TiO_2$, ce dernier pouvant se présenter soit sous forme anatase, soit sous forme rutile, ou bien sous la forme d'un mélange des deux, ou encore sous forme amorphe.

Un tel matériau convient aussi bien pour la synthèse de catalyseurs à base de cobalt qu'à base de fer ou encore de ruthénium ou de nickel. On peut ajouter des métaux des groupes 7, 8, 9 et 10. Un catalyseur à base de cobalt préparé sur ce nouveau support permet en outre d'accroître l'activité tout en gardant une sélectivité en $C_{5+}$ très élevée.

Enfin, un tel matériau permet de réaliser un catalyseur pour la réaction de Fischer-Tropsch économiquement avantageux car la capacité de recyclage des métaux et du support est supérieure à celle connue par l'homme de l'art.

Un premier objet de l'invention est donc un procédé catalytique de conversion au moins partielle d'un mélange gazeux contenant du CO et du $H_2$ en mélange d'hydrocarbures, tel que défini dans la revendication 1.

Ledit support peut se présenter notamment sous la forme de grains, billes, extrudés ou sous la forme de mousse alvéolaire, et dans ce dernier cas notamment sous la forme de plaques ou cylindres.

Dans un mode de réalisation, ladite phase active comprend majoritairement du cobalt ou du fer, et optionnellement l'autre des deux métaux, et optionnellement un ou plusieurs métaux de transition des groupes 7, 8, 9 et/ou 10. Elle peut comprendre également un promoteur, de préférence à une teneur ne dépassant pas 2% massiques. Ledit promoteur peut être sélectionné dans le groupe formé par : ruthénium (promoteur préféré), platine, rhénium, rhodium, iridium, palladium, les terres rares et leurs oxydes, les éléments alcalino-terreux et leurs oxydes.

Dans un autre mode de réalisation, qui peut être combiné avec les autres, ledit support poreux présente une surface spécifique BET supérieure à 5 m$^2$/g, préférentiellement supérieure à 30 m$^2$/g, plus préférentiellement supérieure à 40 m$^2$/g et encore plus préférentiellement supérieure à 60 m$^2$/g.

Dans un autre mode de mode de réalisation, qui peut lui aussi être combiné avec les autres, ledit support poreux présente une surface microporeuse supérieure à 10 m$^2$/g, et de préférence supérieure à 20 m$^2$/g. Avantageusement, son volume poreux, développé dans des pores de diamètres compris entre 30 nm et 300 nm, et mesuré par intrusion de mercure, est supérieur à 0,12 cm$^3$/g, de préférence supérieur à 0,15 cm$^3$/g et encore plus préférentiellement supérieur à 0,20 cm$^3$/g.

Un aspect particulier de l'invention est la réalisation et l'utilisation d'un catalyseur pour la synthèse Fischer-Tropsch qui comprend du cobalt déposé sur un support mixte comportant, d'une part, du $\beta$-SiC et, d'autre part, du TiC et/ou du TiO$_2$, ledit support ayant une surface spécifique supérieure à 30 m$^2$/g, de préférence supérieure à 40 m$^2$/g, et plus particulièrement supérieure à 60 m$^2$/g.

On appelle ici le composite non-oxydé comportant du SiC et du TiC « composite SiC/TiC », et le composite comportant du SiC et du TiO$_2$ « composite SiC/TiO$_2$ », sachant que sauf mention contraire et sauf contexte particulier, dans le composite SiC/TiO$_2$ une faible partie du titane pourra être présente sous la forme de carbure ; l'expression « SiC/TiO$_2$/(TiC) » est ici parfois utilisée pour désigner un composite dans lequel le TiC a été incomplètement oxydé en TiO$_2$.

Préférentiellement le support comporte au moins 0,5% de Ti (notamment sous la forme de TiC et / ou TiO$_2$) et préférentiellement plus de 1%, ces pourcentages étant des pourcentages molaires par rapport à la somme Ti + Si du support ; ils ne prennent pas en compte la phase active. Une teneur molaire en titane comprise entre 0,5% et 15% (et de préférence entre 1% et 10%) est préférée.

Dans un mode de réalisation particulier, on prépare un composite SiC/TiO$_2$ présentant une surface spécifique BET supérieure à 60 m$^2$/g et un volume poreux mesuré par intrusion de mercure supérieur à 0,12 cm$^3$/g pour des pores d'un diamètre compris entre 30 nm et 300 nm. Ce composite est utilisable comme support pour un catalyseur de la synthèse Fischer-Tropsch. Un tel composite peut être préparé par exemple en déposant une source organique de titane (i.e. un précurseur de TiO$_2$) sur un support poreux en SiC, sans passer par la phase TiC. Ce dépôt peut prendre la forme d'une couche, continue ou non : il est ensuite converti en TiO$_2$.

Préférentiellement, la charge de phase active (par exemple la charge de cobalt métallique) est comprise entre 1 et 50% massiques par rapport à la masse totale du catalyseur (cette masse totale correspondant à la somme des masses du support et de la phase active déposée sur ce support), et plus particulièrement entre 5% et 35%, et encore plus préférentiellement entre 5% et 30%.

Avantageusement, le catalyseur comporte au moins un promoteur, de préférence jusqu'à 2% massiques. Le promoteur peut être le ruthénium, le platine, le rhénium, le rhodium, l'iridium, le palladium. On préfère le ruthénium ; avantageusement sa teneur ne dépasse pas les 2%. Le promoteur peut être également choisi parmi les terres rares, les oxydes de terres rares, les éléments alcalino-terreux et leurs oxydes, ainsi que les métaux de transition et leurs oxydes. A titre d'exemple, on peut utiliser du ZrO$_2$ et/ou un oxyde de manganèse.

L'invention concerne également l'utilisation d'un catalyseur pour la réaction de Fischer-Tropsch à base de cobalt supporté sur un composite poreux SiC/TiC, SiC/TiO$_2$ ou SiC/TiO$_2$/(TiC) ayant une surface spécifique supérieure à 30 m$^2$/g, de préférence supérieure à 40 m$^2$/g, et plus particulièrement supérieure à 60 m$^2$/g, avantageusement avec de larges pores.

Dans la méthode selon la revendication 1, on utilise un support de catalyseur dont la méthode de fabrication comprend la préparation d'un mélange comportant au moins une source de silicium, au moins une source de carbone et au moins une source de titane et éventuellement des liants et agents de mise en forme, cette préparation étant suivie d'au moins un traitement thermique qui a pour objet de transformer ladite source de silicium au moins partiellement en carbure de silicium et au moins une partie de ladite source de titane en carbure de titane. Avantageusement, ce traitement thermique est au moins en partie effectué à une température comprise entre 1200°C et 1450°C.

De manière optionnelle, une étape d'oxydation additionnelle à une température d'au moins 350°C, et préférentiellement d'au moins 400°C peut être réalisée pour transformer partiellement ou totalement le carbure de titane en dioxyde de titane. Un tel matériau SiC/TiO$_2$/(TiC) présente en outre une excellente résistance mécanique. Sa surface spécifique peut être supérieure ou égale à 60 m$^2$/g, avec de larges pores.

Encore un autre objet est un procédé de préparation et d'activation d'un catalyseur pour utilisation dans le procédé catalytique de conversion selon l'invention, ledit procédé de préparation et d'activation est défini dans la revendication 13.

Ladite phase de dépôt de précurseur comprend au moins une phase d'imprégnation dudit support avec une solution d'un précurseur de phase active, suivie d'une étape de séchage et d'une étape de calcination.

Ladite calcination se fait avantageusement à une température comprise entre 250°C et 450°C pendant 1 à 14 heures, de préférence entre 300°C et 400°C pendant 4 à 16 heures.

L'invention vise également l'utilisation du catalyseur selon la présente invention pour la réaction de Fischer-Tropsch et qui consiste à convertir un mélange de $H_2$ et de CO (syngas ou gaz de synthèse) majoritairement en hydrocarbures $C_{5+}$.

**Figures**

Les figures 2 à 4 illustrent des modes de réalisation de l'invention, mais ne la limitent pas.

La figure 1 montre une micrographie obtenue par microscopie électronique à balayage d'un échantillon de support $\beta$-SiC poreux selon l'état de la technique, avant imprégnation. La tension d'accélération était de 3 kV, la barre blanche en bas à droite indique la longueur de 1 $\mu$m.

La figure 2 montre une micrographie obtenue par microscopie électronique à balayage d'un échantillon de support $\beta$-SiC/TiC poreux selon l'invention, avant imprégnation. La tension d'accélération était de 3 kV, la barre blanche en bas à droite indique la longueur de 100 $\mu$m.

La figure 3 montre la distribution poreuse obtenue par sorptométrie à l'azote pour un support en $\beta$-SiC poreux connu, pour un support en SiC/TiC selon l'invention, et pour un support en SiC/TiO$_2$ selon l'invention.

La figure 4 montre la distribution du volume poreux (en cm$^3$/g) obtenue par intrusion du mercure en fonction du diamètre de pores (en nanomètres) pour un support en $\beta$-SiC poreux connu et pour un support en TiO$_2$/SiC selon l'invention.

**Description détaillée de l'invention**

D'une manière générale, dans le présent document, le terme « surface spécifique » fait référence à la surface spécifique dite BET, mesurée à l'aide de la méthode de Brunauer, Emmet et Teller, bien connue de l'homme du métier.

La « porosité » d'un matériau est habituellement définie par référence à trois catégories de pores qui se distinguent par leur taille : la microporosité (diamètre inférieur à 2 nm), la mésoporosité (diamètre compris entre 2 et 50 nm) et la macroporosité (diamètre supérieur à 50 nm), voir à ce sujet l'article de F. Rouquerol et al., « Texture des matériaux pulvérulents ou poreux », paru dans la collection Techniques de l'Ingénieur, vol. P 1050.

Sauf mention contraire, tous les pourcentages qui caractérisent une composition chimique sont des pourcentages massiques.

A) Préparation du support

Nous décrivons ici un mode de préparation typique du support. On approvisionne une source de silicium finement divisé, tel que du silicium métallique (toute source de silicium peut convenir) sous la forme d'une poudre, et au moins une source de carbone ou précurseur de carbone. Dans un mode de réalisation, cette source de carbone ou ce précurseur de carbone agit comme un liant lors du procédé de fabrication ; il peut s'agir d'une résine carbonisable. On forme un mélange de la source de silicium et de la source de carbone. On ajoute à ce mélange une source de titane, telle que du TiO$_2$ en poudre (toutes les sources de TiO$_2$ peuvent convenir). On peut ajouter des porogènes pour générer des méso et/ou macropores. Le mélange ainsi obtenu est homogénéisé par les techniques connues de l'homme de l'art.

A ce mélange peut être ajouté un liant temporaire, tel que de l'eau, de l'alcool polyvinylique (PVA), du polyéthylèneglycol (PEG) ou tout autre liant connu de l'homme de l'art. Des agents de dispersion tels que des dispersants ou des agents de peptisation peuvent être ajoutés (par exemple avant ou après introduire la poudre de TiO$_2$ dans le mélange) afin de mieux disperser la poudre de TiO$_2$. Ensuite, ce mélange est mis en forme, par exemple par atomisation et séchage de gouttelettes, ou par extrusion pour obtenir des cylindres, polylobes ou d'autres formes. Si l'extrusion est préférée, des additifs tels que des plastifiants peuvent être ajoutés pour conférer au mélange une consistance qui facilite l'extrusion. Ces plastifiants sont avantageusement carbonisables.

Dans une variante du procédé, on infiltre ce mélange dans une mousse poreuse d'un polymère carbonisable (telle qu'une mousse polyuréthane) ; cette variante permet notamment la préparation de mousses alvéolaires de SiC/TiC/(TiO$_2$).

Après l'étape de mise en forme, le précurseur séché subit un cycle thermique sous atmosphère inerte à une température inférieure à 1450°C (de préférence inférieure à 1400°C) et pendant au moins une heure. On obtient ainsi un composé mixte comportant une phase carbure de titane et une phase carbure de silicium beta ($\beta$-SiC). De manière optionnelle, ce support peut être traité sous air à une température comprise entre 350°C et 500°C pendant 2 à 10 h pour oxyder, totalement ou partiellement, le TiC en TiO$_2$.

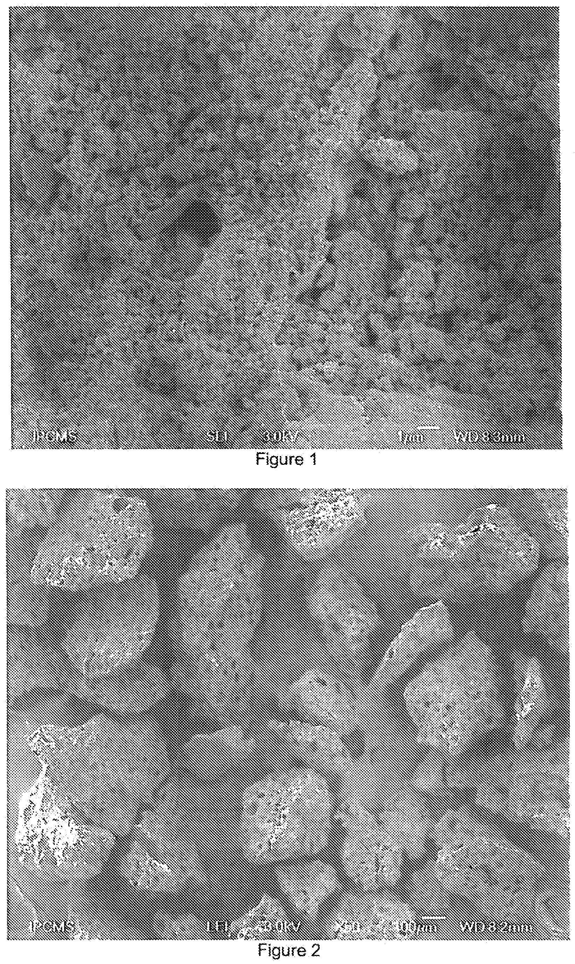

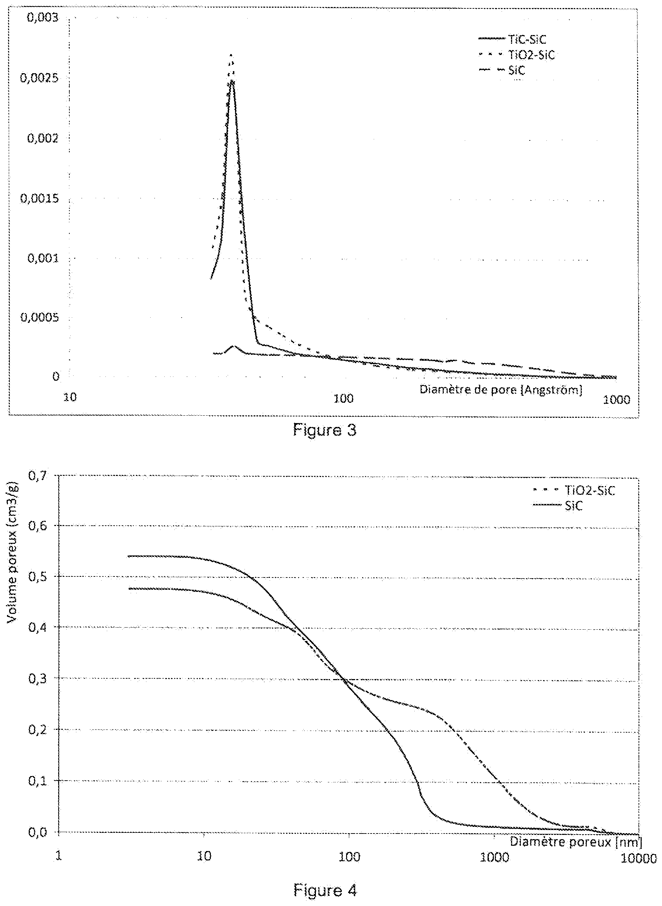

Le support selon l'invention comporte à la fois des micro-, méso- et macropores. La figure 2 montre un cliché obtenu par microscopie électronique à balayage d'un support selon l'invention avant imprégnation. Les macropores sont bien visibles et présentent un diamètre poreux supérieur au micron. Les figures 3 et 4 caractérisent la porosité de quelques supports typiques correspondant à l'état de la technique ($\beta$-SiC) et à la présente invention.

B) Préparation et activation du catalyseur

Nous décrivons ici des procédés pour la préparation et l'activation du catalyseur. En partant d'un support de catalyseur selon l'invention comme décrit ci-dessus, un catalyseur selon l'invention peut être préparé par n'importe quelle technique connue de l'homme du métier. Une technique avantageuse d'imprégnation des métaux et promoteurs est celle de l'imprégnation du volume poreux (*incipient wetness impregnation*). Elle consiste à dissoudre les métaux et promoteurs dans un volume de solvant approximativement égal au volume poreux du support, et à imprégner le support par cette solution.

Le précurseur de cobalt préféré est le nitrate de cobalt, qui est soluble dans l'eau et d'autres solvants alcooliques. Mais d'autres sels ou composés de cobalt peuvent également convenir, par exemple l'acétate de cobalt, le chlorure de cobalt et le carbonyle de cobalt.

Si on souhaite ajouter un promoteur, ce dernier peut être déposé par exemple par co-imprégnation (c'est-à-dire qu'on introduit un de ses sels solubles dans la solution qui comporte le sel du cobalt), ou encore par une deuxième imprégnation (avec une solution d'un sel approprié) qui suit l'étape d'imprégnation du cobalt. Dans le cas où l'on procède par une deuxième imprégnation, on préfère d'abord décomposer le sel (de préférence le nitrate) de cobalt par un traitement thermique, avant de procéder à la seconde imprégnation introduisant le promoteur. Mais on peut également procéder à la réduction de l'oxyde de cobalt en cobalt métallique avant de procéder à ladite seconde imprégnation.

Le promoteur préféré est le ruthénium. Son précurseur peut être un sel de ruthénium capable d'être dissout soit dans une solution aqueuse soit dans une solution organique. On préfère le nitrate de ruthénium ; d'autres sels tels que le chlorure de ruthénium ou l'acétylacétonate de ruthénium peuvent être utilisés.

Parmi les solvants organiques qui conviennent, à la fois pour le sel de cobalt (notamment pour le nitrate de cobalt) et pour le sel du promoteur (et notamment pour le sel de ruthénium, et plus particulièrement pour le nitrate de ruthénium), on peut citer l'acétone, le méthanol, l'éthanol, le diméthyle formamide, le diéthyl éther, le cyclohexane, le xylène et le tetrahydrofurane.

Après l'imprégnation le solide est séché à température ambiante, typiquement pendant 10 heures, puis à une température plus élevée, avantageusement comprise entre 100 et 130°C (typiquement à 110°C) pendant plusieurs heures. Si des solvants organiques sont utilisés, une évaporation lente à l'évaporateur rotatif est préférée. Le catalyseur séché est calciné, de préférence sous air, à une température comprise entre 200°C et 500°C, préférablement entre 200°C et 350°C. Dans un mode de réalisation avantageux, la montée en température se fait à une vitesse comprise entre 0,5°C/min et 5°C/min. La durée du traitement peut être comprise entre 1 et 24 heures et préférablement entre 2 et 6 heures.

Avant son utilisation, le catalyseur doit être activé. Cela peut se faire par réduction sous flux d'hydrogène. Cette activation peut se faire à une température comprise entre 250°C et 450°C, plus particulièrement entre 300°C et 400°C pendant 1 à 24 heures et plus particulièrement entre 4 et 16 heures. Elle peut se faire *in situ* dans le réacteur Fischer-Tropsch. Lors de cette réduction, les éléments métalliques (y compris les promoteurs), qui se trouvent après calcination de leurs précurseurs en général à l'état oxydé, sont réduits en une forme métallique, finement divisée sur la surface poreuse du support, pour former la phase dite active du catalyseur.

C) Utilisation du catalyseur dans la synthèse Fischer-Tropsch

Pour la synthèse Fischer-Tropsch, le catalyseur selon l'invention peut être mis en oeuvre dans les réacteurs connus de l'homme du métier pour cette synthèse, et notamment dans un réacteur à lit fixe multitubulaire et dans un réacteur à lit circulant bouillonnant. On peut utiliser le catalyseur notamment sous la forme de billes (avantageusement des microbilles d'un diamètre compris entre 20 $\mu$m et 400 $\mu$m) ou d'extrudés ou sous la forme de mousse alvéolaire.

D) Avantages de l'invention

Un premier avantage du support selon l'invention est qu'il permet d'accroitre considérablement l'activité du catalyseur sans altérer sa sélectivité en $C_{5+}$. Un autre avantage de ce support et catalyseur est sa remarquable résistance mécanique, sa résistance hydrothermale et sa résistance aux attaques chimiques. Sa haute résistance à l'attrition est particulièrement avantageuse lors de la mise en oeuvre de ce support et catalyseur en réacteur de type « slurry ». Un autre avantage de ce catalyseur est sa stabilité sous flux. Enfin, un autre avantage de ce catalyseur est la possibilité de faciliter la récupération de la phase active et le/les promoteurs du catalyseur usagé ; en effet, ce nouveau support résiste très bien aux traitements acides ou basiques humides qui sont utilisés pour récupérer les éléments métalliques de la phase active. Le composite SiC/TiC et/ou SiC/TiO$_2$ permet, grâce à sa haute surface spécifique, de mieux disperser les particules de cobalt, augmentant de ce fait la productivité du catalyseur. La présence de mésopores d'un diamètre supérieur à environ 30 nm et de macropores d'un diamètre supérieur à 500 nm, ou même supérieur à 1 $\mu$m permet

d'éliminer les phénomènes de diffusion (ou au moins de réduire fortement les limitations diffusionnelles), ce qui conduit à une augmentation de la sélectivité $C_{5+}$.

Le catalyseur selon l'invention est également avantageux pour les procédés lit fixe, car la forte tenue mécanique des grains de catalyseurs permet de limiter la formation de fines lors des chargements et déchargements des réacteurs.

Un catalyseur préparé sur ce nouveau type de support permet en outre d'accroitre l'activité, d'améliorer significativement la sélectivité en $C_{5+}$, et de supporter les contraintes hydrothermales de la synthèse Fischer-Tropsch. Les inventeurs ont constaté que l'utilisation du composite selon l'invention peut permettre de diminuer la charge en phase active lors de la synthèse Fischer-Tropsch. A titre exemple, de bons résultats ont été obtenus avec une charge de cobalt de l'ordre de 10% massiques, alors que les procédés selon l'état de la technique utilisent des catalyseurs avec une charge de cobalt supérieure à 30%, qui peut atteindre 40% ou même 45%.

**Exemples**

Certains aspects de ces exemples sont illustrés par les figures 1 à 4. Dans ces exemples, la surface spécifique a été déterminée à partir des isothermes d'adsorption d'azote à pression variable, et à la température de l'azote liquide, à l'aide d'un appareil automatique Micromeritics Tristar 3000 ™. La surface spécifique totale (appelée « surface spécifique BET ») a été obtenue par la méthode BET, bien connue de l'homme du métier. La surface externe a été obtenue par la méthode t-plot. La surface microporeuse a été obtenue par la différence entre la surface spécifique BET totale et la surface externe. Les distributions micro- et méso-poreuses ont été obtenues à partir de l'isotherme de désorption d'azote. Les distributions méso- et macro-poreuses et le volume poreux total ont été obtenus par intrusion de mercure sur un porosimètre automatique Micrometrics Autopore III ™ type 9420.

Exemple 1 : Préparation du catalyseur de référence 30% Co sur un support de SiC

Une masse de 20 g de support de catalyseur en carbure de silicium beta ($\beta$-SiC) de surface spécifique moyenne (26 $m^2$/g), voir la figure 1, a été imprégnée avec 42,32 g de nitrate de cobalt dissous dans 20 g d'eau distillée. Après imprégnation, le solide a été séché à température ambiante pendant 12 heures puis étuvé à 110°C pendant 2 heures. Ensuite le solide a été calciné sous air à 350°C pendant 2 heures. L'oxyde de cobalt ainsi obtenu a été réduit sous 300 cc/min d'hydrogène à 300°C pendant 6 heures. Le catalyseur a été passivé à température ambiante sous un flux contenant 1 vol.% d'$O_2$ dilué dans de l'hélium.

Exemple 2 : Préparation d'un catalyseur 30% Co sur un support de SiC/$TiO_2$

Une masse de 20 g de support de catalyseur en SiC/$TiO_2$ (voir la figure 2) présentant une micro, méso et macroporosité et ayant une surface spécifique de 86 $m^2$/g a été imprégnée avec 42,32 g de nitrate de cobalt dissous dans 20 g d'eau distillée. Après imprégnation, le solide a été séché à température ambiante pendant 12 heures puis étuvé à 110°C pendant 2 heures. Ensuite le solide a été calciné sous air à 350°C pendant 2 heures. L'oxyde de cobalt ainsi obtenu a été réduit sous 300 $cm^3$/min d'hydrogène à 300°C pendant 6 heures. Le catalyseur a été passivé à température ambiante sous un flux contenant 1 vol.% d'$O_2$ dilué dans de l'hélium.

Exemple 3 : Préparation d'un catalyseur 10% Co sur un support de SiC/$TiO_2$

Une masse de 20 g de support de catalyseur en SiC/$TiO_2$ présentant un micro, méso et macroporosité et ayant une surface spécifique de 86 $m^2$/g a été imprégnée avec 10,97 g de nitrate de cobalt dissous dans 20 g d'eau distillée. Après imprégnation, le solide a été séché à température ambiante pendant 12 heures puis étuvé à 110°C pendant 2 heures. Ensuite le solide a été calciné sous air à 350°C pendant 2 heures. L'oxyde de cobalt ainsi obtenu a été réduit sous 300 cc/min d'hydrogène à 300°C pendant 6 heures. Le catalyseur a été passivé à température ambiante sous un flux contenant 1 vol.% d'$O_2$ dilué dans de l'hélium.

Exemple 4 : Préparation d'un catalyseur 10% Co sur un support de SiC/TiC

Une masse de 20 g de support de catalyseur en TiC/SiC présentant une micro, méso et macroporosité et ayant une surface spécifique de 72 $m^2$/g a été imprégnée avec 10,97 g de nitrate de cobalt dissous dans 20 g d'eau distillée. Après imprégnation, le solide a été séché à température ambiante pendant 12 heures puis étuvé à 110°C pendant 2 heures. Ensuite le solide a été calciné sous air à 350°C pendant 2 heures. L'oxyde de cobalt ainsi obtenu a été réduit sous 300 cc/min d'hydrogène à 300°C pendant 6 heures. Le catalyseur a été passivé à température ambiante sous un flux contenant 1 vol.% d'$O_2$ dilué dans de l'hélium.

Exemple 5 : Activité en Synthèse Fischer-Tropsch

Les catalyseurs décrits dans les exemples 1 à 4 ont été testés en synthèse de Fischer-Tropsch. 5 g de catalyseur sous forme de grains de 250-400 $\mu$m de diamètre ont été placés dans un réacteur en acier inoxydable ayant un diamètre de 6 mm. La pression du système a été augmentée jusqu'à 4 MPa (avec une rampe de 4 MPa.h$^{-1}$) sous un flux d'argon. Lorsque la pression désirée a été atteinte, on a augmenté la température du réacteur jusqu'à 210°C (rampe de chauffe de 2°C.min$^{-1}$). Lorsque la température désirée a été atteinte, on a remplacé le flux d'argon par un mélange 50 :50 v:v d'argon et de gaz de synthèse ($H_2$/CO, 2 :1 v :v). Le catalyseur a été activé pendant 3 jours sous flux dilué avant d'être soumis à un mélange réactionnel de $H_2$/CO pur, la température du réacteur pouvait alors être variée. Les activités et sélectivités des catalyseurs des exemples 1 à 4 à différentes températures et vitesses spatiales horaires sont consignées dans les tableaux 1 à 3. L'activité d'un catalyseur à base de SiC/TiC est plus que doublée comparée à celle d'un catalyseur à base de SiC. Le surcroît d'activité peut être mesuré dans le réacteur par une élévation de la température plus élevée pour le catalyseur selon l'invention.

Tableau 1 : Activité et sélectivité en synthèse Fischer-Tropsch

| | GHSV [h$^{-1}$] | CO conversion [%] | | $C_{5+}$ [% massiques] | | $g_{C5+}/g_{catalyst}/h$ | |
| --- | --- | --- | --- | --- | --- | --- | --- |
| | | T=215°C | T=220°C | T=215°C | T=220°C | T=215°C | T=220°C |
| Exemple 1 (30%Co sur SiC) | 1900 | 43,37 | 57,38 | 91 | 90,23 | 0,23 | 0,26 |
| Exemple 2 30%Co sur SiC/TiO$_2$ | 2750 | 58,78 | 68,23 | 92,42 | 93,08 | 0,41 | 0,48 |

Tableau 2 : Activité et sélectivité en synthèse Fischer-Tropsch

| | GHSV [h$^{-1}$] | CO conversion [%] | | $c_{5+}$ [% massiques] | | $g_{C5+}/g_{catalyst}/h$ | |
| --- | --- | --- | --- | --- | --- | --- | --- |
| | | T=225°C | T=227°C | T=225°C | T=227°C | T=225°C | T=227°C |
| Exemple 1 (30% Co sur SiC) | 1900 | 71,23 | - | 90,28 | - | 0,32 | - |
| Exemple 2 (30% Co sur SiC/TiO$_2$) | 3800 | 58,61 | 62,49 | 92,32 | 90,84 | 0,54 | 0,57 |

Tableau 3 : Activité et sélectivité en synthèse Fischer-Tropsch

| | GHSV [h$^{-1}$] | CO conversion [%] | $C_{5+}$ [% massiques] | $g_{C5+}/g_{catalyst}/h$ |
| --- | --- | --- | --- | --- |
| | | T = 215°C | T = 215°C | T = 215°C |
| Exemple 3 (10% Co sur SiC/TlO$_2$) | 2750 | 36 | 93,49 | 0,17 |
| Exemple 4 (10% Co sur SiC/TiC) | 2750 | 53,05 | 91,54 | 0,24 |

Tableau 4 : Données physiques des supports

| | Surface spécifique BET [m$^2$/g] | Surface spécifique due aux micropores [m$^2$/g] | Surface spécifique externe [m$^2$/g] | Volume poreux cumulé obtenu par intrusion au mercure [cm$^3$/g] |
| --- | --- | --- | --- | --- |
| SiC | 25,6 | 1,2 | 24,4 | 0,52 |
| SiC/TiC | 72 | 43 | 29 | 0,43 |
| SiC/TiO$_2$ | 86 | 50 | 36 | 0,43 |

**Revendications**

**1.** Procédé catalytique de conversion au moins partielle d'un mélange gazeux contenant du CO et du $H_2$ en mélange

d'hydrocarbures, comportant une étape de mise en contact dudit mélange gazeux avec un catalyseur solide, ledit catalyseur solide comportant

- un support poreux comportant un matériau composite comportant du SiC et un carbure de titane (composite appelé « SiC/TiC ») et/ou un oxyde de titane (composite appelé « SiC/TiO$_2$ »), et
- une phase active, ledit matériau composite ayant été préparé par une méthode comprenant la préparation d'un mélange comportant au moins une source de silicium, au moins une source de carbone et au moins une source de titane et éventuellement des liants et agents de mise en forme, cette préparation étant suivie d'au moins un traitement thermique qui a pour objet de transformer ladite source de silicium au moins partiellement en carbure de silicium et au moins une partie de ladite source de titane en carbure de titane.

2. Procédé selon la revendication 1, dans lequel ledit traitement thermique a été au moins en partie effectué à une température comprise entre 1200°C et 1450°C.

3. Procédé selon la revendication 1 ou 2, dans lequel ladite méthode de préparation dudit matériau composite comprend en plus une étape d'oxydation additionnelle à une température d'au moins 350°C, et préférentiellement d'au moins 400°C, pour transformer partiellement ou totalement le carbure de titane en dioxyde de titane.

4. Procédé selon l'une quelconque des revendications 1 à 3, dans lequel la teneur en phase active par rapport à la masse totale dudit support poreux avec sa phase active est comprise entre 1 à 50% massiques, de préférence entre 5% et 35% et encore plus préférentiellement entre 5% et 30%.

5. Procédé selon l'une quelconque des revendications 1 à 4, dans lequel ladite phase active comprend majoritairement du cobalt ou du fer, et optionnellement l'autre des deux métaux, et optionnellement un ou plusieurs métaux de transition des groupes 7, 8, 9 et/ou 10.

6. Procédé selon l'une quelconque des revendications 1 à 5, dans lequel ladite phase active comprend un promoteur, de préférence à une teneur ne dépassant pas 2% massiques.

7. Procédé selon la revendication 6, **caractérisé en ce que** ledit promoteur est sélectionné dans le groupe formé par : ruthénium (promoteur préféré), platine, rhénium, rhodium, iridium, palladium, les terres rares et leurs oxydes, les éléments alcalino-terreux et leurs oxydes, les métaux de transition et leurs oxydes.

8. Procédé selon l'une quelconque des revendications 1 à 7, dans lequel ledit support poreux présente une surface spécifique BET supérieure à 5 m$^2$/g, préférentiellement supérieure à 30 m$^2$/g, plus préférentiellement supérieure à 40 m$^2$/g et encore plus préférentiellement supérieure à 60 m$^2$/g.

9. Procédé selon l'une quelconque des revendications 1 à 8, dans lequel ledit support poreux présente une teneur molaire en titane par rapport à la somme molaire Si + Ti comprise entre 0,5% et 15%, et de préférence entre 0,5% et 10%.

10. Procédé selon l'une quelconque des revendications 1 à 9, dans lequel ledit support poreux présente une surface microporeuse supérieure à 10 m$^2$/g, et de préférence supérieure à 20 m$^2$/g.

11. Procédé selon la revendication 10, **caractérisé en ce que** ledit support poreux présente un volume poreux dans la gamme 30 nm à 300 nm mesuré par intrusion de mercure supérieur à 0,12 cm$^3$/g, de préférence supérieure à 0,15 cm$^3$/g et encore plus préférentiellement supérieur à 0,20 cm$^3$/g.

12. Procédé selon l'une quelconque des revendications 1 à 11, **caractérisé en ce que** ledit support se présente sous la forme de grains, de billes, d'extrudés, ou sous la forme de mousse alvéolaire.

13. Procédé de préparation et d'activation d'un catalyseur pour utilisation dans le procédé catalytique de conversion selon l'une quelconque des revendications 1 à 12, ledit procédé de préparation et d'activation comprenant :

(a) au moins une étape de dépôt de précurseur de phase active sur un support poreux comportant un matériau composite de type SiC/TiC et/ou de type SiC/TiO$_2$ ;
(b) au moins une phase d'activation dudit précurseur de phase active pour former la phase active,

sachant que ledit matériau composite de type SiC/TiC et/ou de type SiC/TiO$_2$ mis en oeuvre à l'étape (a) a été préparé par une méthode comprenant la préparation d'un mélange comportant au moins une source de silicium, au moins une source de carbone et au moins une source de titane et éventuellement des liants et agents de mise en forme, cette préparation ayant été suivie d'au moins un traitement thermique qui a pour objet de transformer ladite source de silicium au moins partiellement en carbure de silicium et au moins une partie de ladite source de titane en carbure de titane.

14. Procédé selon la revendication 13, **caractérisé en ce que** ladite phase de dépôt de précurseur comprend au moins une phase d'imprégnation dudit support avec une solution d'un précurseur de phase active, suivie d'une étape de séchage et d'une étape de calcination.

15. Procédé selon la revendication 14, **caractérisé en ce que** ladite calcination se fait à une température comprise entre 250°C et 450°C pendant 1 à 14 heures, de préférence entre 300°C et 400°C pendant 4 à 16 heures.

**Patentansprüche**

1. Katalytisches Verfahren zur mindestens teilweisen Umwandlung einer CO und H$_2$ enthaltenden Gasmischung in eine Kohlenwasserstoffmischung, umfassend einen Schritt des Inkontaktbringens der Gasmischung mit einem festen Katalysator, wobei der feste Katalysator umfasst:

   - einen porösen Träger umfassend ein Verbundmaterial, das SiC und ein Titancarbid (Verbundmaterial mit der Bezeichnung "SiC / TiC") und / oder ein Titanoxid (Verbundmaterial mit der Bezeichnung "SiC / TiO$_2$") umfasst, und
   - eine aktive Phase,
   wobei besagtes Verbundmaterial durch ein Verfahren hergestellt wurde, das das Zubereiten einer Mischung umfasst, die mindestens eine Siliziumquelle, mindestens eine Kohlenstoffquelle und mindestens eine Titanquelle und gegebenenfalls Bindemittel und Formgebungsmittel umfast, wobei auf dieses Zubereiten mindestens eine Wärmebehandlung folgt, die die Siliziumquelle zumindest teilweise in Siliziumkarbid und die Titanquelle mindestens teilweise in Titancarbid umwandeln soll.

2. Verfahren nach Anspruch 1, in dem besagte Wärmebehandlung zumindest teilweise bei einer Temperatur zwischen 1200 ° C und 1450 ° C durchgeführt wurde.

3. Verfahren nach Anspruch 1 oder 2, in dem besagtes Verfahren zur Herstellung besagten Verbundmaterials ferner einen zusätzlichen Oxidationsschritt bei einer Temperatur von mindestens 350 ° C und vorzugsweise mindestens 400 ° C umfasst, um das Titancarbid teilweise oder vollständig in Titandioxid umzuwandeln

4. Verfahren nach irgendeinem der Ansprüche 1 bis 3, bei dem der Gehalt an aktiver Phase im Bezug auf die Gesamtmasse des porösen Trägers mit seiner aktiven Phase zwischen 1 und 50 Gew.-%, vorzugsweise zwischen 5 % und 35 %, und noch bevorzugter zwischen 5 % und 30 % liegt.

5. Verfahren nach irgendeinem der Ansprüche 1 bis 4, bei dem besagte aktive Phase vorwiegend Kobalt oder Eisen und gegebenenfalls das andere der beiden Metalle, und gegebenenfalls ein oder mehrere Übergangsmetalle der Gruppen 7, 8, 9 und 10 umfasst.

6. Verfahren nach irgendeinem der Ansprüche 1 bis 5, bei dem besagte aktive Phase einen Promotor umfasst, vorzugsweise mit einem Gehalt von nicht mehr als 2 Gew.-%.

7. Verfahren nach Anspruch 6, **dadurch gekennzeichnet, dass** besagter Promotor ausgewählt ist aus der Gruppe bestehend aus: Ruthenium (bevorzugter Promotor), Platin, Rhenium, Rhodium, Iridium, Palladium, den seltenen Erden und ihren Oxiden, den Erdalkalielementen und ihren Oxiden, den Übergangsmetalle und ihren Oxiden.

8. Verfahren nach irgendeinem der Ansprüche 1 bis 7, bei dem besagter poröser Träger eine spezifische BET-Oberfläche von mehr als 5 m$^2$/g, vorzugsweise mehr als 30 m$^2$/g, besonders bevorzugt mehr als 40 m$^2$/g, und noch bevorzugter mehr als 60 m$^2$/g aufweist.

9. Verfahren nach irgendeinem der Ansprüche 1 bis 8, wobei besagter poröser Träger einen molaren Gehalt an Titan in Bezug auf den molaren Si + Ti - Gehalt zwischen 0,5% und 15% und vorzugsweise zwischen 0,5% und 10% aufweist.

10. Verfahren nach irgendeinem der Ansprüche 1 bis 9, wobei besagter poröser Träger eine mikroporöse Oberfläche von mehr als 10 m$^2$/g und vorzugsweise mehr als 20 m$^2$/g aufweist.

11. Verfahren nach Anspruch 10, **dadurch gekennzeichnet, dass** besagter poröser Träger ein Porenvolumen im Bereich von 30 nm bis 300 nm aufweist, gemessen durch Quecksilber-Intrusion, das grösser ist als 0,12 cm$^3$/g, vorzugsweise grösser als 0,15 cm$^3$/g und noch bevorzugter größer als 0,20 cm$^3$/g.

12. Verfahren nach irgendeinem der Ansprüche 1 bis 11, **dadurch gekennzeichnet, dass** besagter Träger in Form von Körnern, Perlen, Extrusionen oder in Form von Zellschaum vorliegt.

13. Verfahren zur Herstellung und Aktivierung eines Katalysators zur Verwendung in einem katalytischen Umwandlungsverfahren nach irgendeinem der Ansprüche 1 bis 12, wobei das besagte Herstellungs- und Aktivierungsverfahren umfasst:

(a) mindestens einen Abscheidungsschritt von Vorstufe einer aktiven Phase auf einem porösen Träger, der ein Verbundmaterial vom SiC / TiC-Typ und / oder vom SiC / TiO$_2$ -Typ umfasst;
(b) mindestens eine Aktivierungsphase des besagten Vorläufers der aktiven Phase, um die aktive Phase zu bilden,

wobei besagtes Verbundmaterial vom SiC / TiC-Typ und / oder vom SiC / TiO$_2$ - Typ durch ein Verfahren hergestellt wurde, das das Zubereiten einer Mischung umfasst, die mindestens eine Siliziumquelle, mindestens eine Kohlenstoffquelle und mindestens eine Titanquelle und gegebenenfalls Bindemittel und Formgebungsmittel umfast, wobei auf dieses Zubereiten mindestens eine Wärmebehandlung folgt, die die Siliziumquelle zumindest teilweise in Siliziumkarbid und die Titanquelle mindestens teilweise in Titancarbid umwandeln soll.

14. Verfahren nach Anspruch 13, **dadurch gekennzeichnet, dass** der Abscheidungsschritt des Vorläufers mindestens eine Imprägnierungsphase des Trägers mit einer Lösung eines Vorläufers der aktiven Phase umfasst, gefolgt von einem Trocknungsschritt und einem Schritt des Kalzinierens.

15. Verfahren nach Anspruch 14, **dadurch gekennzeichnet, dass** besagtes Kalzinieren 1 bis 14 Stunden bei einer Temperatur zwischen 250 °C und 450 °C, vorzugsweise 4 bis 16 Stunden bei einer Temperatur zwischen 300 °C und 400 °C durchgeführt wird.

## Claims

1. Catalytic process for at least partial conversion of a gaseous mixture containing CO and H$_2$ into a hydrocarbon mixture, comprising a step of bringing said gaseous mixture into contact with a solid catalyst, said solid catalyst comprising :

- a porous support comprising a composite material comprising SiC and a titanium carbide (composite called "SiC / TiC") and / or a titanium oxide (composite called "SiC / TiO$_2$"), and
- an active phase,
wherein said composite material has been prepared by a method comprising preparing a mixture comprising at least one silicon source, at least one carbon source and at least one titanium source and optionally binders and shaping agents, this preparation being followed by at least one heat treatment which is intended to transform said silicon source at least partially into silicon carbide and at least a part of said titanium source into titanium carbide.

2. Process according to claim 1, wherein said the heat treatment has been at least partly carried out at a temperature of between 1200 ° C and 1450 ° C.

3. Process according to claim 1 or 2, wherein said method of preparing said composite material further comprises an additional oxidation step at a temperature of at least 350 ° C, and preferably at least 400 ° C, to partially or totally transform titanium carbide into titanium dioxide.

4.  Process according to any one of claims 1 to 3, in which the content of active phase with respect to the total mass of said porous support with its active phase is between 1 and 50% by weight, preferably between 5% and 35% and even more preferably between 5% and 30%.

5.  Process according to any one of claims 1 to 4, wherein said active phase comprises predominantly cobalt or iron, and optionally the other of the two metals, and optionally one or more transition metals of groups 7, 8, 9 and / or 10.

6.  Process according to any one of claims 1 to 5, wherein said active phase comprises a promoter, preferably at a content not exceeding 2% by weight.

7.  Process according to claim 6, **characterized in that** said promoter is selected from the group consisting of: ruthenium (preferred promoter), platinum, rhenium, rhodium, iridium, palladium, the rare elements earths and their oxides, the alkaline earth elements and their oxides, the transition metals and their oxides.

8.  Process according to any one of claims 1 to 7, in which said porous support has a BET specific surface area greater than 5 $m^2$/g, preferably greater than 30 $m^2$/g, more preferably greater than 40 $m^2$/g, and even more preferably greater than 60 $m^2$/g.

9.  Process according to any one of claims 1 to 8, wherein said porous support has a molar content of titanium with respect to the molar Si + Ti content of between 0.5% and 15%, and preferably between 0.5% and 10%.

10. A process according to any one of claims 1 to 9, wherein said porous support has a microporous surface greater than 10 $m^2$/g, and preferably greater than 20 $m^2$/g.

11. Process according to claim 10, **characterized in that** said porous support has a pore volume in the range 30 nm to 300 nm measured by mercury intrusion greater than 0.12 $cm^3$/g, preferably greater than 0.15 $cm^3$/g and even more preferably greater than 0.20 $cm^3$/g.

12. Process according to any one of claims 1 to 11, **characterized in that** said support is in the form of grains, beads, extrusions, or in the form of cellular foam.

13. Process for preparing and activating a catalyst for use in the catalytic conversion process according to any one of claims 1 to 12, said preparation and activation process comprising:

    (a) at least one deposition step of precursor of active phase on a porous support comprising a composite material of SiC / TiC type and / or of SiC / $TiO_2$ type;
    (b) at least one activation step of said precursor of active phase to form the active phase,
    wherein said composite material of SiC / TiC type and / or of SiC / $TiO_2$ type used in step (a) has been prepared by a method comprising the preparation of a mixture comprising at least one silicon source, at least one carbon source and at least one titanium source and optionally binders and shaping agents, this preparation being followed by at least one heat treatment which is intended to transform said silicon source at least partially into silicon carbide and at least a part of said titanium source into titanium carbide.

14. Process according to claim 13, **characterized in that** said precursor deposition step comprises at least one impregnation step of said support with a solution of a precursor of active phase, followed by a drying step and a calcination step.

15. Process according to claim 14, **characterized in that** said calcination is carried out at a temperature of between 250 ° C and 450 ° C for 1 to 14 hours, preferably between 300 ° C and 400 ° C for 4 to 16 hours.

Figure 1

Figure 2

Figure 3

Figure 4

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6875720 B
- US 20050124490 A
- US 7163963 B
- US 5484757 A
- US 7351679 B
- US 5648312 A

**Littérature non-brevet citée dans la description**

- *Journal of Catalysis,* 2005, vol. 236, 139-152
- *Journal of Catalysis,* 2008, vol. 259, 161-164
- *Applied Catalysis A : General,* 2010, vol. 154, 162-182
- Selectivity control and catalyst design in the Fischer-Tropsch synthesis : sites, pellets and reactors. *Advances in Catalysis,* 1993, vol. 39, 221-302